# EUROPEAN PATENT SPECIFICATION

(11) **EP 1 336 657 B1**
(45) Date of publication and mention of the grant of the patent: **25.06.2008**
(21) Application number: 01982863.1
(22) Date of filing: 20.11.2001
(51) Int. Cl.: C12N 15/54, C12N 1/21, C12P 7/66, C12N 9/12

(54) **PROCESS FOR PRODUCING COENZYME Q 10**
VERFAHREN ZUR HERSTELLUNG VON COENZYM Q 10
PROCEDE SERVANT A PREPARER UN COENZYME Q 10

(30) Priority: 20.11.2000 JP 2000352940
(43) Date of publication of application: 20.08.2003
(73) Proprietor: KANEKA CORPORATION, Osaka-shi, Osaka 530-8288 (JP)
(72) Inventor: MATSUDA, Hideyuki, Matsue-shi, Shimane 690-0815 (JP); KAWAMUKAI, Makoto, Matsue-shi, Shimane 690-0823 (JP); YAJIMA, Kazuyoshi, Akashi-shi, Hyogo 673-0005 (JP); IKENAKA, Yasuhiro, Kobe-shi, Hyogo 651-2242 (JP)
(74) Representative: HOFFMANN EITLE
(86) International application number: PCT/JP2001/010119
(87) International publication number: WO 2002/040682

(56) References cited:
- EP-A- 1 123 979
- EP-A2- 0 812 914
- JP-A- 10 057 072
- JP-A- 11 178 590
- US-A- 4 220 719
- US-A- 4 367 289
- US-A- 6 103 488
- T. KOYAMA ET AL.: 'Thermostable farnesyl diphosphate synthase of bacillus stearothermophilus: molecular cloning, sequence determination, overproduction and purification' J. BIOCHEM. vol. 113, no. 3, 1993, pages 355 - 363, XP000561783
- M. TADA: 'Mechanism of photoregulated carotenogenesis in rhodotorula minuta VI. photocontrol of ubiquinone production' PLANT CELL PHYSIOL. vol. 30, no. 8, 1989, pages 1193 - 1196, XP002909357

## Description

### TECHNICAL FIELD

The present invention relates to a process for producing coenzyme Q₁₀, which is in use as a drug or the like. More particularly, it relates to a process for causing formation of coenzyme Q₁₀ by isolating a gene coding for a coenzyme Q₁₀ side chain synthase, which serves as a key enzyme in the biosynthesis of coenzyme Q₁₀, namely a decaprenyl diphosphate synthase, from a fungal species belonging to the genus *Rhodotorula* and introducing that gene into a host microorganism.

### BACKGROUND ART

An industrial process for producing coenzyme Q₁₀, which is conventional in the art, comprises, for example, isolating coenzymes Q of plant origin, for example of tobacco origin, and adjusting the side chain length thereof by a synthetic method.

It is known that coenzyme Q₁₀ is produced in a wide variety of organisms, from microorganisms, such as bacteria and yeasts, to higher animals and plants. Thus, the process comprising cultivating a microorganism and extracting this substance from cells thereof can be regarded as one of the most efficient process for producing Q₁₀ and has actually been employed in commercial production thereof. However, the productivity of such processes can hardly be said to be good, since the yield is low and the procedure is complicated, for instance.

As for analogs (e.g. coenzyme Q₈) differing in chain length from coenzyme Q₁₀, not Q₁₀ itself, attempts have also been made to increase the production thereof by isolating genes involved in the biosynthesis thereof and amplifying the genes utilizing the recombinant DNA technology.

Coenzyme Q₁₀ is formed *in vivo* in a multistage process comprising complicated reactions in which a number of enzymes are involved. The route of biosynthesis thereof in prokaryotes partially differs from that in eukaryotes. Basically, however, each route comprises three fundamental steps, namely the step of synthesis of decaprenyl diphosphate, which is the source of the prenyl side chain of coenzyme Q₁₀, the step of synthesis of para-hydroxybenzoic acid, which is the source of the quinone ring, and the step of completion of coenzyme Q₁₀ through coupling of these two compounds and successive substituent conversions. Among these reactions, the reaction which determines the side chain length of coenzyme Q₁₀, namely the decaprenyl diphosphate synthase-involving reaction, which is said to be a rate-determining one in the whole biosynthetic reaction route, is considered to be the most important one.

Therefore, for efficient production of coenzyme Q₁₀, it is considered effective to isolate a decaprenyl diphosphate synthase gene, which is the key gene in the biosynthesis of coenzyme Q₁₀, and utilize the same for the purpose of increasing production. As for the gene source, fungi, in which coenzyme Q₁₀ is produced in relatively large amounts, are leading candidates.

So far, decaprenyl diphosphate synthase genes have been isolated from several microorganisms, such as *Schizosacharomyces pombe* (JP-A-09-173076) and *Gluconobacter suboxydans* (JP-A-10-57072). However, the productivity of coenzyme Q₁₀ in these microorganisms cannot be said to be satisfactory, and the cultivation of these microorganisms and the separation/purification of coenzyme Q₁₀ therefrom have not been efficient. It has thus been desired that a microorganism-derived gene for that enzyme, which enables high level production of coenzyme Q₁₀, be isolated.

From EP 1 123 979 a process for producing coenzyme Q₁₀ is known. A gene coding for the enzyme synthesizing coenzyme Q₁₀ side chain synthase from a fungal strain of the genus Saitoella is isolated and used for the microbial production of coenzyme Q₁₀.

From US 6,103,488, a process for producing ubiquinone-10 is known. A gene coding for decaprenyl diphospate synthase from photosynthetic bacterium *Rhodobacter capsulatus* is isolated and used for the microbial production of coenzyme Q10.

From EP 0 812 914, a technique for cloning prenyl diphosphate synthetase gene of *Micrococcus luteus* B-P 26, and a technique for preparing a probe for the purpose of cloning are known.

From JP-11-178590 A, a process for producing coenzyme Q₁₀ is known. A gene coding for decaprenyl diphospate synthase from phptosynthetic bacterium *Paracoccus denitrificans* is cloned and used for the microbial production of ubiquinone-10.

From JP-10-57072 A, a process for producing ubiquinone-10 is known. A gene coding for decaprenyl diphospate synthase from phptosynthetic bacterium *Gluconobacter suboxydans* is isolated and used for the microbial production of coenzyme Q₁₀.

From Koyama T. et al., J. Biochem. (1993), Vol. 113, 355, it is known that the decaprenyl diphosphate synthase has 7 regions in which the amino acid sequence has been relatively preserved beyond the difference in enzymes or Organism species.

US 4,220,719 discloses that various species of microorganism of the genus Rhodotorula produce coenzyme Q₁₀. US 4,220,719 does not specify the genes that perform the synthesis of coenzyme Q10 from microorganisms of the genus Rhodotorula.

### SUMMARY OF THE INVENTION

It is an object of the present invention to produce coenzyme Q₁₀ efficiently in microorganisms by isolating a coenzyme Q₁₀ side chain synthesis gene from a fungal species belonging to the genus *Rhodotorula* and utilizing the same to thereby solve the above-mentioned productivity problem.

For attaining the above object, the present inventors made investigations in an attempt to isolate a decaprenyl diphosphate synthase gene from fungi belonging to the genus *Rhodotorula,* in which coenzyme Q₁₀ is produced in relatively large amounts, and succeeded in isolating such gene. As a result of further investigations made by them to increase the expression level of that gene, they succeeded in improving the gene so that coenzyme Q₁₀ may be expressed more abundantly. These successes have now led to completion of the present invention.

Thus, the present invention provides a DNA improved in its expression in prokaryotes, namely
a DNA of the following (d), (e) or (f):
(d) a DNA whose base sequence is as described under SEQ ID NO:3;
(e) a DNA having a DNA sequence derived from the base sequence shown under SEQ ID NO:3 by deletion, addition, insertion and/or substitution of one base and
   encoding a protein having decaprenyl diphosphate synthase activity;
(f) a DNA capable of hybridizing with a DNA comprising the base sequence shown under SEQ ID NO:3 under stringent conditions and
   encoding a protein having decaprenyl diphosphate synthase activity.

The invention further provides a DNA improved in its expression in prokaryotes, namely
a DNA of the following (k), (l) or (m):
(k) a DNA whose base sequence is as described under SEQ ID NO:5;
(l) a DNA having a DNAsequence derived from the base sequence shown under SEQ ID NO:5 by deletion, addition, insertion and/or substitution of one base and
   encoding a protein having decaprenyl diphosphate synthase activity;
(m) a DNA capable of hybridizing with a DNA comprising the base sequence shown under SEQ ID NO:5 under stringent conditions and
   encoding a protein having decaprenyl diphosphate synthase activity.

The invention further provides
a protein of the following (i) or (j):
(i) a protein whose amino acid sequence is as described under SEQ ID NO:4;
(j) a protein having an amino acid sequence derived from the amino acid sequence shown under SEQ ID NO:4 by deletion, addition, insertion and/or substitution of one amino acid residue and having decaprenyl diphosphate synthase activity.

The invention further provides
a protein of the following (n) or (o):
(n) a protein whose amino acid sequence is as described under SEQ ID NO:6;
(o) a protein having an amino acid sequence derived from the amino acid sequence shown under SEQ ID NO:6 by deletion, addition, insertion and/or substitution of one amino acid residue and having decaprenyl diphosphate synthase activity.

The invention further provides
DNAs respectively encoding the above proteins (i) to (j).

The invention further provides
expression vectors with the above DNAs inserted into vectors.

The invention further provides
transformants resulting from transformation of host microorganisms with the respective DNAs mentioned above or with the expression vectors mentioned above.

The invention further provides
a DNA encoding the protein (n) or (o).

The invention further provides
expression vectors with the above DNA inserted into vectors.

The invention further provides
transformants resulting from transformation of host microorganisms with the respective DNAs mentioned above or with the expression vectors mentioned above.

The invention still further provides
a process for producing coenzyme Q₁₀,
which comprises cultivating any of the above-mentioned transformants in a medium and recovering coenzyme Q₁₀ thus formed and accumulated in the medium.

### DETAILED DISCLOSURE OF THE INVENTION

In the following, the present invention is described in detail.

The present inventors made investigations to isolate a gene encoding the enzyme in question from fungi belonging to the genus *Rhodotorula,* in which coenzyme Q₁₀ is produced in relatively large amounts, and, as a result, succeeded in obtaining a fragment of the gene by the PCR method.

The sequence of a known gene encoding a decaprenyl diphosphate synthase was compared with that of a known gene encoding a polyprenyl diphosphate synthase which is an analogue with different chain length of the enzyme in question and a long prenyl chain synthase of coenzyme Q, to the decaprenyl diphosphate synthase, and various PCR primers were synthesized for regions showing high homology therebetween. PCR conditions were studied for various combinations of these primers and, as a result, it was revealed, by gene base sequence analysis, that when 40 PCR cycles, each comprising 94°C, 1 minute → 43°C, 2 minute → 72°C, 2 minutes, are carried out after 3 minutes of heat treatment at 94°C, using the primers DPS-1 (5'-AAGGATCCTNYTNCAYGAYGAYGT-3') and DPS-1 1AS (5'-ARYTGNADRAAYTCNCC-3') (in these sequences, R representing A or G, Y representing C or T, and N representing G, A, T or C), a fragment, about 220 bp in size, of the enzyme gene in question is amplified from the chromosomal gene of *Rhodotorula minuta* IFO 0387, which is a fungal species belonging to the genus *Rhodotorula.*

For obtaining the enzyme gene in its full length, the chromosomal gene of *Rhodotorula minuta* IFO 0387 was cleaved with the restriction enzyme *Eco*RI, and the cleavage products were inserted into a λ phage vector to construct a recombinant phage library. The resulting plaques were transferred to a nylon membrane, and plaque hybridization was carried out using the PCR fragment in a labeled form, whereby a clone having the decaprenyl diphosphate synthase gene in its full length could be obtained.

The base sequence of the decaprenyl diphosphate synthase gene contained in the clone obtained was determined, whereupon it was revealed that it has the sequence shown under SEQ ID NO:1 in the sequence listing. In the amino acid sequence (amino acid sequence described under SEQ ID NO:2 in the sequence listing) predicted from this base sequence, there was found a sequence characteristic of decaprenyl diphosphate synthase.

Since, in eukaryotes, the decaprenyl diphosphate synthase gene is expressed and functions in the mitochondria, it is supposed that, in the sequence on the amino acid terminal side of this gene sequence, there be a sequence allowing localization thereof in the mitochondria. Therefore, the inventors considered that, for more effective functioning of this gene in prokaryotes, it be necessary to specify and eliminate that or those sequences which is or are not essential in prokaryotes. As a result of studies on the amino acid terminal side sequence of that gene sequence, it has become possible to produce coenzyme Q₁₀ in significant amounts by using the gene specified under SEQ ID NO:3. The amino acid sequence deducible from the DNA sequence shown under SEQ ID NO:3 is described under SEQ ID NO:4 in the sequence listing. As a result of further studies on the amino acid terminal side sequence, it has become possible to produce coenzyme Q₁₀ in significant amounts by using the gene specified under SEQ ID NO:5. The amino acid sequence deducible from the DNA sequence shown under SEQ ID NO:5 is described under SEQ ID NO:6 in the sequence listing.

The DNA of the present invention may be a DNA whose base sequence is as described under SEQ ID NO:3, or a DNA having a base sequence derived from the base sequence shown under SEQ ID NO:3 by deletion, addition, insertion and/or substitution of one base and encoding a protein having decaprenyl diphosphate synthase activity, or a DNA capable of hybridizing with a DNA comprising the base sequence shown under SEQ ID NO:3 under stringent conditions and encoding a protein having decaprenyl diphosphate synthase activity. A number of amino acids each may be encoded by one or more codons (genetic code degeneracy), so that a number of DNAs other than the DNA having the base sequence shown under SEQ ID NO:3 can encode the protein having the amino acid sequence shown under SEQ ID NO:4. Therefore, the DNA of the invention includes such DNAs encoding the protein having the amino acid sequence shown under SEQ ID NO:4 as well.

The DNA of the present invention may be a DNA whose base sequence is as described under SEQ ID NO:5, or a DNA having a base sequence derived from the base sequence shown under SEQ ID NO:5 by deletion, addition, insertion and/or substitution of one base and encoding a protein having decaprenyl diphosphate synthase activity, or a DNA capable of hybridizing with a DNA comprising the base sequence shown under SEQ ID NO:5 under stringent conditions and encoding a protein having decaprenyl diphosphate synthase activity. A number of amino acids each may be encoded by one or more codons (genetic code degeneracy), so that a number of DNAs other than the DNA having the base sequence shown under SEQ ID NO:5 can encode the protein having the amino acid sequence shown under SEQ ID NO:6. Therefore, the DNA of the invention includes such DNAs encoding the protein having the amino acid sequence shown under SEQ ID NO:6 as well.

The expression "base sequence derived by deletion, addition, insertion and/or substitution of one base" as used herein means a base sequence resulting from deletion, addition, insertion and/or substitution of such one base as can be deleted, added, inserted and/or substituted according to the methods well known to those skilled in the art, for example those described in Supplemental issue, Tanpakushitsu, Kakusan, Koso (Protein, Nucleic Acid and Enzyme), PCR Method for Gene Amplification, TAKKAJ, 35 (17), 2951-3178 (1990) or Henry A. Erlich (ed.), translated into Japanese under the supervision of Ikunoshin Kato: PCR Technology (1990).

The expression "DNA capable of hybridizing with a DNA comprising the base sequence shown under SEQ ID NO:3 (or SEQ ID NO:5) under stringent conditions" means a DNA obtainable by utilizing the technique of colony hybridization, plaque hybridization or southern hybridization, among others, using a DNA comprising the base sequence shown under SEQ ID NO:3 (or SEQ ID NO:5) as a probe. Those skilled in the art would be able to readily obtain the desired DNA by carrying out such hybridization according to the method described in Molecular Cloning, 2nd edition (Cold Spring Harbor Laboratry Press, 1989).

The expression "protein having decaprenyl diphosphate synthase activity" means a protein capable of synthesizing decaprenyl diphosphate in a yield of not less than 10%, preferably not less than 40%, more preferably not less than 60%, still more preferably not less than 80%, as compared with the case where a protein having the amino acid sequence shown under, SEQ ID NO:4 or SEQ ID NO:6. Such capacity can be measured by reacting FPP (farnesyl diphosphate) with ¹⁴C-IPP (radiolabeled isopentenyl diphosphate) in the presence of the enzyme in question, hydrolyzing the resulting ¹⁴C-DPP (decaprenyl diphosphate) with phosphatase and, after separation by TLC, determining the incorporation in each spot for each chain length (Okada et al., Eur. J. Biochem., 255, 52-59).

The protein of the invention may be a protein whose amino acid sequence is as described under SEQ ID NO:4 or SEQ ID NO:6, or a protein having an amino acid sequence derived from the amino acid sequence shown under, SEQ ID NO:4 or SEQ ID NO:6 by.deletion, addition, insertion and/or substitution of one amino acid residue and having decaprenyl diphosphate synthase activity.

"Such an amino acid sequence derived by deletion, addition, insertion and/or substitution of one amino acid residues" can be obtained by deleting, adding, inserting and/or substituting one amino acid residue or residues by site-specific mutagenesis or any other methods well known in the art. Such methods are specifically described, for example, in Nucleic Acid Res., 10, 6487 (1982) and Methods in Enzymology, 100, 448 (1983).

For causing expression of the decaprenyl diphosphate synthase gene, it is necessary to connect that gene to a site downstream of an appropriate promoter. It is possible to construct an expression vector, for example, by excising a DNA fragment containing the gene using a restriction enzyme or amplifying an enzyme-encoding gene portion alone by PCR and then inserting the fragment or amplification product into a promoter-containing vector.

In the practice of the invention, the vector in which a DNA encoding a protein having decaprenyl diphosphate synthase activity is to be inserted to give an expression vector is not particularly restricted but may be one derived from an *Escherichia coli*-derived plasmid with an appropriate promoter inserted therein. The *Escherichia coli*-derived plasmid includes, among others, pBR322, pBR325, pUC19, and pUC119, and the promoter includes, among others, the T7 promoter, trp promoter, tac promoter, lac promoter, and λPL promoter. In the practice of the invention, pGEX-2T, pGEX-3T, pGEX-3X (the three being products of Pharmacia), pBluescriptII, pUC19, pUC18 (product of Toyobo Co., Ltd.), pMALC2, pET-3T, pUCNT (described in WO 94/03613) and the like may also be used as vectors for expression. Among these, pUCNT is judiciously used. In specific examples, a decaprenyl diphosphate synthase gene expression vector, pNTRm2, can be constructed by inserting a DNA comprising the base sequence shown under SEQ ID NO:1 into the vector for expression pUCNT, and an expression vector, pNTRm6, can be constructed by inserting a DNA comprising the base sequence shown under SEQ ID NO:3 into pUCNT. An expression vector, pUCRm3, can be constructed when a DNA comprising the base sequence shown under SEQ ID NO:5 is inserted into the vector for expression pUC18.

And, by introducing the above enzyme gene expression vector into an appropriate microorganism, it becomes possible to utilize the microorganism for the production of coenzyme Q₁₀. The host microorganism is not particularly restricted but *Escherichia coli* is judiciously used. The *Escherichia coli* strain is not particularly restricted but includes XL1-Blue, BL-21, JM109, NM522, DH5α, HB101, DH5, and pUC18, among others. Among these, *Escherichia coli* HB101 and pUC18 are judiciously used. For example, when the decaprenyl diphosphate synthase gene expression vector pNTRm2, pNTRm6 or pUCRm3 is introduced into *Escherichia coli*, this can be transformed so that coenzyme Q₁₀, which *Escherichia coli* originally does not produce, can be produced in significant amounts. The *Escherichia coli* strain with pNTRm2 introduced therein has been deposited, under the Budapest Treaty, with the National Institute of Advanced Industrial Science and Technology International Patent Organism Depositary (Central 6, 1-1-1 Higashi, Tsukuba, Ibaraki, Japan) under the designation *E. coli* HB101(pNTRm2) FERM BP-7333, the *Escherichia coli* strain with pNTRm6 introduced therein under *E. coli* HB101(pNTRm6) FERM BP-7332, and the *Escherichia coli* strain with pUCRm3 under *E. coli* DH5α(pUCRm3) FERM BP-7638.

The gene of the invention may be used singly, or may be introduced into a microorganism together with another gene or other genes involved in the biosynthesis of coenzyme Q₁₀ for expression thereof. In the latter case, better results can be expected.

Coenzyme Q₁₀ can be produced by cultivating the transformant obtained in the invention in a medium in the conventional manner and recovering coenzyme Q₁₀ from the cultivation product. In cases where the host microorganism is *Escherichia coli,* LB medium, or M9 medium containing glucose and casamino acids can be used as the medium. For better promoter functioning, such an agent as isopropylthiogalactoside or indolyl-3-acrylic acid, for instance, may be added to the medium. The cultivation is carried out, for example, at 37°C for 17 to 24 hours, if necessary with aeration and/or agitation. In the practice of the invention, the product coenzyme Q₁₀ obtained may be purified or used in the form of a crude product according to the selection duly made depending on the intended use thereof. Coenzyme Q₁₀ can be isolated from the cultivation product by an appropriate combination of per se known methods of separation and/or purification. The per se known methods of separation and/or purification include salting out, solvent precipitation and other methods utilizing the difference in solubility, dialysis, ultrafiltration, gel filtration, (SDS-)polyacrylamide gel electrophoresis and other methods mainly utilizing the difference in molecular weight, ion exchange chromatography and other methods utilizing the difference in charge, affinity chromatography and other methods utilizing specific affinity, reversed phase high-performance liquid chromatography and other methods utilizing the difference in hydrophobicity, isoelectric focusing and other methods utilizing the difference in isoelectric point, among others.

The field of utilization of coenzyme Q₁₀ obtained in the present invention is not particularly restricted but it may be judiciously used as a drug, among others.

### BRIEF DESCRIPTION OF THE DRAWINGS

Fig. 1 is a restriction enzyme map of the expression vector pNTRm2.
Fig. 2 is a restriction enzyme map of the expression vector pNTRm6.
Fig. 3 shows HPLC analysis charts for the host and transformant products.

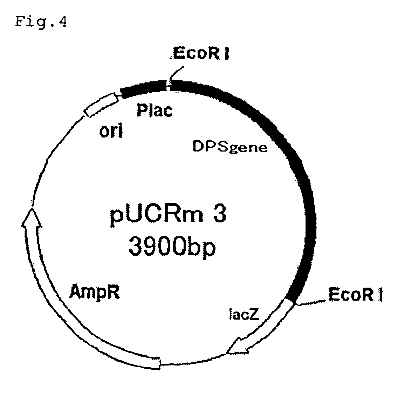

Fig. 4 is a restriction enzyme map of the expression vector pUCRm3.
Fig. 5 shows HPLC analysis charts for the products in the host for the expression vector pUCRm3 and in the transformant.

### BEST MODES FOR CARRYING OUT THE INVENTION

The following examples illustrate the present invention in more detail. These examples are, however, by no means limitative of the scope of the invention.

### (Example 1)

The chromosomal DNA of *Rhodotorula minuta* IFO 0387 was prepared by the method of C. S. Hoffman et al. (Gene, 57 (1987), 267-272). Based on the homology with the known long-chain prenyl diphosphate synthase gene, primers for use in PCR, namely DPS-1 (5'-AAGGATCCTNYTNCAYGAYGAYGT-3') and DPS-1 1AS (5'-ARYTGNADRAAYTCNCC-3'), were designed. In these sequences, R represents A or G, Y represents C or T, and N represents G, A, T or C. Using these, a PCR cycle of 94°C, 1 minute → 43°C, 2 minutes → 72°C, 2 minutes, were repeated 40 times after 3 minutes of heat treatment at 94°C (ExTaq, product of Takara, being used as the enzyme), followed by 1.2% agarose gel electrophoresis.

The thus-obtained fragment, about 220 bp in size, was purified by excising the corresponding gel portion from the gel and then treating with a DNA extraction kit (Sephaglas (trademark) BrandPrep Kit, product of Amersham Pharmacia Biotech), and the purified fragment was cloned into a vector for expression in *Escherichia coli* using a PCR product direct cloning kit (pT7 BlueT-Vector Kit, product of NOVAGEN) to give pT7-RmDPS. The DNA base sequence was determined by carrying out the reaction on a DNA sequencer (model 377, product of PerkinElmer) using a DNA sequencing kit (product of PerkinElmer, ABI PRISM (trademark) BigDye (trademark) Terminator Cycle Sequence Ready Reaction Kit with AmptiTaq (registered trademark) DNA polymerase, FS) and according to the manual attached thereto. As a result, a sequence covering the 823rd to 1029th bases of the base sequence shown under SEQ ID NO:1 in the sequence listing was obtained. The sequence "GDFLLARA", which is a characteristic region of the long-chain prenyl chain-containing prenyl diphosphate synthase, could be found in the sequence translated from the base sequence determined in the above manner. It was therefore estimated that the sequence obtained be part of the decaprenyl diphosphate synthase gene.

### (Example 2)

Using 0.03 µg of the pT7-RmDPS vector DNA having the 220 bp DNA fragment supposed to be part of the decaprenyl diphosphate synthase gene of *Rhodotorula minuta* IFO 0387 and using the PCR primers Rm-1S (having the sequence 5'-GCCATGAGGAGAGCACAAGCG-3') and Rm-2AS (having the sequence 5'-CACGGAGGCTACTAGCTCGAC-3'), PCR (94°C, 3 minutes → (94°C, 30 seconds → 55°C, 30 seconds → 72°C, 1 minute) x 25 cycles → 72°C, 5 minutes → 4°C) was carried out, followed by 1.2% agarose (product of Takara Shuzo Co., Ltd.) gel electrophoresis. After excision of the corresponding portion from the gel, a fragment, about 145 bp in size, was purified using a DNA extraction kit (Sephaglas (trademark) BrandPrep Kit, product of Amersham Pharmacia Biotech). About 100 ng of this DNA fragment was subjected to chemiluminescence labeling using an ECL direct nucleic acid labeling system (product of Amersham Pharmacia Biotech).

### (Example 3)

The chromosomal DNA of *Rhodotorula minuta* IFO 0387 was cleaved with the restriction enzyme *Eco*RI, followed by 0.8% agarose gel electrophoresis. The gel was denatured with an alkali (0.5 M NaOH, 1.5 M NaCl) and then neutralized (0.5 M Tris-HCl (pH 7.5), 1.5 M NaCl), a Hibond N+ filter (product of Amersham) was laid on the gel, and southern transfer was effected overnight using 20 x SSC. The filter was dried and baked at 80°C for 2 hours and, thereafter, southern hybridization and detection were carried out using an ECL direct nucleic acid labeling/detection system (product of Amersham Pharmacia Biotech). Thus, using a Gold hybridization solution (product of Amersham Pharmacia Biotech), prehybridization was carried out at 42°C for 1 hour.

The chemiluminescence-labeled probe was heated at 95°C for 5 minutes, then quenched in ice, and added to the prehybridization for the prehydridized filter, and hybridization was carried out at 42°C for 22 hours. This filter was washed with two portions of a 0.5 x SSC solution containing 6 M urea and 0.4% SDS at 42°C for 20 minutes (for each portion) and then with two portions of a 2 x SSC solution at room temperature for 5 minutes (for each portion). This filter was immersed in an enhanced chemiluminescence reagent (product of Amersham Pharmacia Biotech) and brought into close contact with an X ray film for exposure thereof, and black bands resulting from exposure were detected. As a result, strong hybridization with a fragment, about 5.5 kbp in size, resulting from the cleavage with the restriction enzyme EcoRI was detected.

### (Example 4)

The chromosomal DNA of *Rhodotorula minuta* IFO 0387 was cleaved with the restriction enzyme *Eco*RI, followed by 0.8% agarose gel electrophoresis. A DNA fragment for use in cloning was prepared by excising the gel portion containing a DNA fragment in the vicinity of about 5.5 kbp in size and purifying the fragment. Using a λ-ZAPII phage kit (product of Stratagene), this DNA fragment was inserted into the phage at its *Eco*RI site, followed by packaging using an *in vitro* packaging kit (product of Amersham). *Escherichia coli* XL1-Blue MRF' was infected with the phage and layered onto an NZY plate medium (5 g/L Nacl, 2 g/L MgSO₄·7H₂O, 5 g/L yeast extract, 10 g/L NZ amine, 18 g/L agar, pH 7.5), together with an NZY soft agar medium (same as the NZY plate medium except for the content of agar, which was 8 g/L), for plaque formation. The plaques were transferred onto a Hibond N+ filter (product of Amersham), denatured with an alkali (0.5 M NaOH, 1.5 M NaCl), neutralized (0.5 M Tris-HCl (pH 7.5), 1.5 M NaCl), dried, and baked at 80°C for 2 hours.

Using 6 filters after baking as prepared in the above manner, prehybridization and hybridization using the chemiluminescence-labeled probe were carried out in the same manner as in Example 3, and the filters were washed. After drying, the filters were brought into close contact with an X ray film for exposure of the same, and the phage plaques corresponding to black spots resulting from exposure were isolated. *Escherichia coli* was infected with the phages of the thus-isolated plaques in the same manner as mentioned above for plaque formation. The plaques obtained were transferred onto a filter and again subjected to hybridization for confirmation. Seven phage strains could be selected.

*Escherichia coli* SOLR in the λ-ZAPII phage kit (product of Stratagene) was infected with each of those phages in a suspension form, together with the helper phage, and a phagemid was prepared *in vitro.* The above phagemid contained an insert fragment of about 5.5 kbp and, when PCR was carried out using the primers Rm-1S and Rm-2AS, the 145 bp DNA fragment could be detected. The DNA base sequence was determined by carrying out the reactions on a DNA sequencer (model 377, product of PerkinElmer, Inc.) using the internal primers Rm-1S and Rm-2AS and using a DNA sequencing kit (product of PerkinElmer, Inc., ABI PRISM (trademark) BigDye (trademark) Terminator Cycle Sequence Ready Reaction Kit with AmptiTaq (registered trademark) DNA polymerase, FS) according to the manual attached thereto. As a result of repeated sequencing using primers prepared based on the sequence revealed by preceding sequencing, the whole sequence of the decaprenyl diphosphate synthase gene of *Rhodotorula minuta* IFO 0387 could be revealed. Thus, the base sequence of a DNA of about 1.6 kbp was determined. The results are shown under SEQ ID NO:1 in the sequence listing. The amino acid sequence deduced from this DNA sequence is shown under SEQ ID NO:2.

### (Example 5)

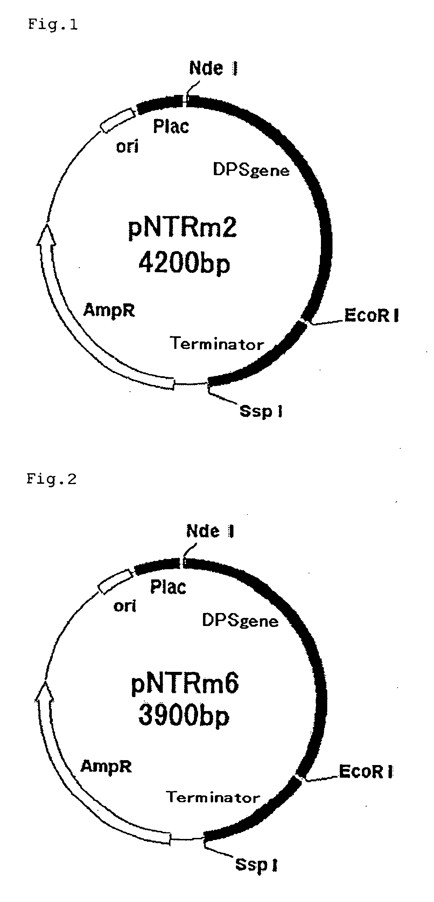

For excising the decaprenyl diphosphate synthase-encoding gene portion from the phagemid DNA prepared, PCR was carried out in the same manner as in Example 2 using two synthetic DNA primers designated RM-1 (having the sequence 5'-ATCATATGATGCACCGACAAGCT-3') and Rm-CE2 (having the sequence 5'-AAGAATTCCTACTTTGTTCGGTTGAGCACAG-3'). The amplification product was cleaved with the restriction enzymes *Nde*I and *Eco*RI*,* and the cleavage product was inserted into a vector for expression, pUCNT (described in WO 94/03613) to give a decaprenyl diphosphate synthase gene expression vector, pNTRm2. The restriction enzyme map of the thus-obtained expression vector pNTRm2 is shown in Fig. 1. The symbol DPS stands for the coding region of the decaprenyl diphosphate synthase gene.

### (Example 6)

The thus-constructed decaprenyl diphosphate synthase gene expression vector pNTRm2 was introduced into *Escherichia coli* HB101, the microorganisms were shake-cultured overnight in 10 mL of LB medium at 37°C, and cells were harvested by centrifugation (3,000 revolutions, 20 minutes).

The cells were suspended in 1 mL of a 3% aqueous solution of sulfuric acid and, after 30 minutes of heat treatment at 120°C, 2 mL of a 14% aqueous solution of sodium hydroxide was added, followed by further 15 minutes of heat treatment at 120°C. To the thus-treated suspension was added 3 mL of hexane-isopropanol (10:2) for effecting extraction. After centrifugation, 1.5 mL of the organic solvent layer was separated, and the solvent was evaporated to dryness under reduced pressure conditions. The residue was dissolved in 200 µl of ethanol, and 20 µl of the solution was analyzed by high-performance liquid chromatography (using LC-10A, product of Shimadzu Corp.). For separation, a reversed phase column (YMC-pack ODS-A, 250 x 4.6 mm, S-5 µm, 120A) was used, together with ethanol-methanol (2:1) as the mobile phase solvent. The coenzyme Q₁₀ formed was detected based on the absorbance at the wavelength 275 nm. The results are shown in Fig. 2.
As shown in Fig. 2, it was revealed that, upon introduction of the decaprenyl diphosphate synthase gene for expression of Q₁₀, the transformant *Escherichia coli* strain was now possible to produce coenzyme Q₁₀, which is originally not produced in *Escherichia coli.*

The thus-obtained recombinant *Escherichia coli* HB101(pNTRm2) has been deposited, under the Budapest Treaty, with the National Institute of Advanced Industrial Science and Technology International Patent Organism Depositary (Central 6, 1-1-1 Higashi, Tsukuba, Ibaraki, Japan) as of October 19, 2000 (deposition/accession No. FERM BP-7333).

### (Example 7)

For eliminating the mitochondrial transitional sequence characteristic of eukaryotes from the sequence under SEQ ID NO:1 in order to attain abundant expression in prokaryotes, PCR was carried out in the same manner as in Example 2 using two synthetic DNA primers designated Rm-4 (having the sequence 5'-ATCATATGAATATTCGACCCACTCCAACT-3') and Rm-CE2 (having the sequence 5'-AAGAATTCCTACTTTGTTCGGTTGAGCACAG-3'). The thus-amplified 1.3 kbp fragment was cleaved with the restriction enzymes *Nde*I and *Nhe*I*,* and the thus-prepared fragment, about 600 bp in size, was recombined to the major fragment of pNTRm2 as obtained by digestion with the restriction enzymes *Nde*I and *Nhe*I to construct pNTRmSsp. Further, PCR was carried out in the same manner as in Example 2 using two synthetic DNA primers designated RM-1 (having the sequence 5'-ATCATATGATGCACCGACAAGCT-3') and RM-6R (having the sequence 5'-ACAATATTGTATTGAGGGCATTCGGGCGACTGC-3') for amplifying a fragment, about 100 bp in size, resulting from deletion of the N portion. The fragment was cleaved with the restriction enzymes *Nde*I and *Ssp*I, and the resulting fragment was recombined to the major fragment of pNTRmSsp as resulting from digestion with the restriction enzymes *Nde*I and *Ssp*I to construct pNTRm6.

### (Example 8)

The thus-constructed decaprenyl diphosphate synthase gene expression vector pNTRm6 was introduced into *Escherichia coli* HB101, the microorganisms were shake-cultured overnight in 10 mL of LB medium at 37°C, and cells were harvested by centrifugation (3,000 revolutions, 20 minutes).

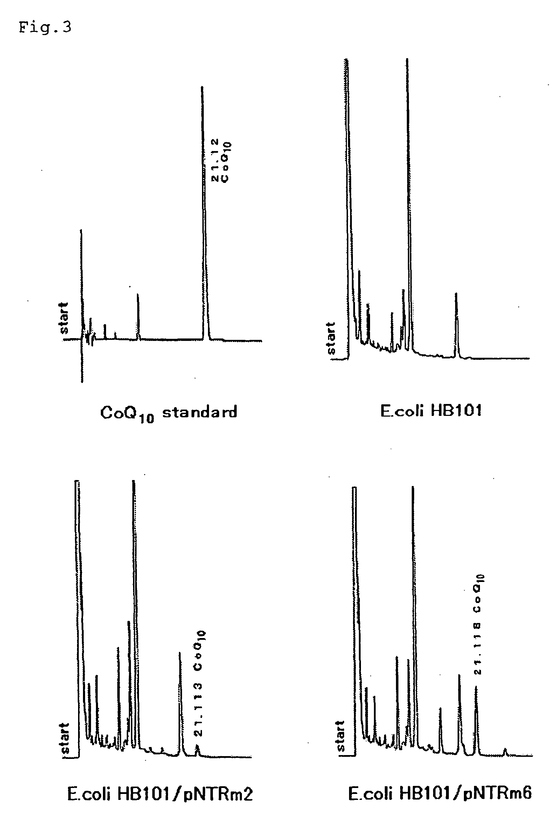

The cells were suspended in 1 mL of a 3% aqueous solution of sulfuric acid and, after 30 minutes of heat treatment at 120°C, 2 mL of a 14% aqueous solution of sodium hydroxide was added, followed by further 15 minutes of heat treatment at 120°C. To the thus-treated suspension was added 3 mL of hexane-isopropanol (10:2) for effecting extraction. After centrifugation, 1.5 mL of the organic solvent layer was separated, and the solvent was evaporated to dryness under reduced pressure conditions. The residue was dissolved in 200 µl of ethanol, and 20 µl of the solution was analyzed by high-performance liquid chromatography (using LC-10A, product of Shimadzu Corp.). For separation, a reversed phase column (YMC-pack ODS-A, 250 x 4.6 mm, S-5 µm, 120A) was used, together with ethanol-methanol (2:1) as the mobile phase solvent. The coenzyme Q₁₀ formed was detected based on the absorbance at the wavelength 275 nm. The results are shown in Fig. 3.
As shown in Fig. 3, it was revealed that, upon introduction of the decaprenyl diphosphate synthase gene for expression thereof, coenzyme Q₁₀, which is originally not produced in *Escherichia coli,* could now be produced in the transformant and that conversion could be attained so as to attain coenzyme Q₁₀ production in significantly larger amounts as compared with the *Escherichia coli* strain transformed with pNTRm2.

The thus-obtained recombinant *Escherichia coli* HB101(pNTRm6) has been deposited, under the Budapest Treaty, with the National Institute of Advanced Industrial Science and Technology International Patent Organism Depositary (Central 6, 1-1-1 Higashi, Tsukuba, Ibaraki, Japan) as of October 19, 2000 (deposition/accession No. FERM BP-7332).

### (Example 9)

For eliminating the mitochondrial transitional sequence characteristic of eukaryotes from the sequence under SEQ ID NO:1 in order to attain abundant expression in prokaryotes, PCR was carried out in the same manner as in Example 2 using two synthetic DNA primers designated RmNEco (having the sequence 5'-ACGAATTCGATGATCTTCGACCCACTCCAACT-3') and Rm-CE2 (having the sequence 5'-AAGAATTCCTACTTTGTTCGGTTGAGCACAG-3'), with pNTRm2 as the template. The thus-amplified 1.2 kbp fragment was cleaved with the restriction enzymes *Bam*HI and *Eco*RI, and the thus-prepared fragment was joined to the major fragment of pUC18 as obtained by digestion with the restriction enzymes *Bam*HI and *Eco*RI to construct pUCRm3.

### (Example 10)

The thus-constructed decaprenyl diphosphate synthase gene expression vector pUCRm3 was introduced into *Escherichia coli* DH5α, the microorganisms were shake-cultured overnight in 10 mL of LB medium at 37°C, and cells were harvested by centrifugation (3,000 revolutions, 20 minutes).

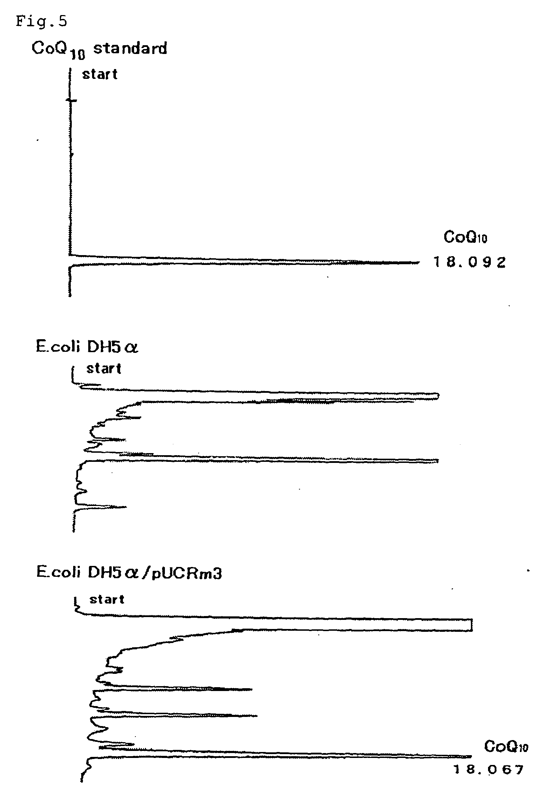

The cells were suspended in 1 mL of a 3% aqueous solution of sulfuric acid and, after 30 minutes of heat treatment at 120°C, 2 mL of a 14% aqueous solution of sodium hydroxide was added, followed by further 15 minutes of heat treatment at 120°C. To the thus-treated suspension was added 3 mL of hexane-isopropanol (10:2) for effecting extraction. After centrifugation, 1.5 mL of the organic solvent layer was separated, and the solvent was evaporated to dryness under reduced pressure conditions. The residue was dissolved in 200 µl of ethanol, and 20 µl of the solution was analyzed by high-performance liquid chromatography (using LC-10A, product of Shimadzu Corp.). For separation, a reversed phase column (YMC-pack ODS-A, 250 x 4.6 mm, S-5 µm, 120A) was used, together with ethanol-methanol (2:1) as the mobile phase solvent. The coenzyme Q₁₀ formed was detected based on the absorbance at the wavelength 275 nm. The results are shown in Fig. 5.
As shown in Fig. 5, it was revealed that, upon introduction of the decaprenyl diphosphate synthase gene for expression thereof, coenzyme Q₁₀, which is originally not produced in *Escherichia coli,* could now be produced in the transformant and that conversion could be attained so as to attain coenzyme Q₁₀ production in significantly larger amounts as compared with the *Escherichia coli* strains transformed with pNTRm2 and pNTRm6, respectively.

The thus-obtained recombinant *Escherichia coli* DH5α(pUCRm3) has been deposited, under the Budapest Treaty, with the National Institute of Advanced Industrial Science and Technology International Patent Organism Depositary (Central 6, 1-1-1 Higashi, Tsukuba, Ibaraki, Japan) as of June 22, 2001 (deposition/accession No. FERM BP-7638).

### INDUSTRIAL APPLICABILITY

A gene encoding decaprenyl diphosphate synthase, which is the key enzyme in the biosynthesis of coenzyme Q₁₀, was isolated from a fungal species belonging to the genus *Rhodotorula* and it was sequenced. This could successfully be introduced in *Escherichia coli* for expression thereof. Furthermore, improvements in gene sequence successfully resulted in production of coenzyme Q₁₀ in significant amounts. By using the method of the invention, it becomes possible to efficiently produce coenzyme Q₁₀, which is in use as a drug, among others.

## Claims

1. A DNA of the following (d), (e) or (f):
(d) a DNA whose base sequence is as described under SEQ ID No: 3;
(e) a DNA having a DNA sequence derived from the base sequence shown under SEQ ID No: 3 by deletion, addition, insertion and/or substitution of one base and encoding a protein having decaprenyl diphosphate synthase activity;
(f) a DNA capable of hybridizing with a DNA comprising the base sequence shown under SEQ ID No: 3 under stringent conditions and
encoding a protein having decaprenyl disphosphate synthase activity.

2. A protein of the following (i) or (j):
(i) a protein whose amino acid is as described under SEQ ID No:4;
(j) a protein having an amino acid sequence derived from the amino acid sequence shown under SEQ ID No:4 by deletion, addition, insertion and/or substitution of one amino acid residue and having decaprenyl diphosphate synthase activity.

3. A DNA encoding the protein according to claim 2.

4. An expression vector resulting from the insertion of the DNA according to claim 1 or 3 into a vector for expression.

5. The expression vector according to claim 4, wherein the vector for expression is pUCNT.

6. The expression vector according to claim 5, which is pNTRm2 (FERM BP-7333).

7. The expression vector according to claim 5, which is pNTRm6 (FERM BP-7332).

8. A transformant resulting from transformation of a host microorganism with the DNA according to claim 1 or 3.

9. A transformant resulting from transformation of a host microorganism with the expression vector according to claim 4, 5, 6 or 7.

10. The transformant according to claim 8 or 9, wherein the host microorganism is a strain of *Escherichia coli.*

11. The transformant according to claim 10, wherein the strain of *Escherichia coli* is *Escherichia coli* HB101.

12. The transformant according to claim 11, which is *E. coli* HB101 (pNTRm2) (FERM BP-7333).

13. The transformant according to claim 11, which is *E. coli* HB101 (pNTRm6) (FERM BP-7332).

14. A process for producing coenzyme Q₁₀ which comprises cultivating the transformant according to claim 8, 9, 10, 11, 12 or 13 in a medium and recovering coenzyme Q₁₀ thus formed and accumulated in the medium.

15. A DNA of the following (k), (l) or (m):
(k) a DNA whose base sequence is as described under SEQ ID No:5;
(l) a DNA having a DNA sequence derived from the base sequence shown under SEQ ID No:5 by deletion, addition, insertion and/or substitution of one base and encoding a protein having decaprenyl diphosphate synthase activity;
(m) a DNA capable of hybridizing with a DNA comprising the base sequence shown under SEQ ID No: 5 under stringent conditions and
encoding a protein having decaprenyl diphosphate synthase activity.

16. A protein of the following (n) or (o):
(n) a protein whose amino acid is as described under SEQ ID No:6;
(o) a protein having an amino acid sequence derived from the amino acid sequence shown under SEQ ID No:6 by deletion, addition, insertion and/or substitution of one amino acid and having decaprenyl diphosphate synthase activity.

17. A DNA encoding the protein according to claim 16.

18. An expression vector resulting from the insertion of the DNA according to claim 15 or 17 into a vector for expression.

19. The expression vector according to claim 18, wherein the vector for expression is pUC18.

20. The expression vector according to claim 19, which is pUCRm3 (FERM BP-7638).

21. A transformant resulting from transformation of a host microorganism with the DNA according to claim 15 or 17.

22. A transformant resulting from transformation of a host microorganism with the expression vector according to claim 18, 19 or 20.

23. The transformant according to claim 21 or 22, wherein the host microorganism is a strain of *Escherichia coli.*

24. The transformant according to claim 23, wherein the strain of *Escherichia coli* is *Escherichia coli* DH5α.

25. The transformant according to claim 24, which is *E. coli* DH5α (pUCRm3) (FERM BP-7638).

26. A process for producing coenzyme Q₁₀ which comprises cultivating the transformant according to claim 21, 22, 23, 24 or 25 in a medium and recovering coenzyme Q₁₀ thus formed and accumulated in the medium.

## Patentansprüche

1. DNA gemäß den folgenden (d), (e) oder (f):
(d) eine DNA mit einer Basensequenz wie beschrieben unter SEQ ID Nr. 3;
(e) eine DNA mit einer DNA-Sequenz abgeleitet von der Basensequenz wie dargestellt unter SEQ ID Nr. 3 durch Deletion, Addition, Insertion und/oder Substitution einer Base und ein Protein mit einer Decaprenyldiphosphat-Synthaseaktivität codierend;
(f) eine DNA, die mit einer DNA hybrisidieren kann, umfassend die Basensequenz wie dargestellt unter SEQ ID Nr. 3 unter stringenten Bedingungen;
und ein Protein mit einer Decaprenyldiphosphat-Synthaseaktivität codierend.

2. Protein gemäß den folgenden (i) oder (j):
(i) ein Protein mit einer Aminosäuresequenz wie beschrieben unter SEQ ID Nr. 4;
(j) ein Protein mit einer Aminosäuresequenz abgeleitet von der Aminosäuresequenz wie dargestellt unter SEQ ID Nr. 4 durch Deletion, Addition, Insertion und/oder Substitution eines Aminosäurerests und mit einer Decaprenyldiphosphat-Synthaseaktivität.

3. DNA, codierend das Protein gemäß Anspruch 2.

4. Expressionsvektor, der sich aus der Insertion der DNA gemäß Anspruch 1 oder 3 in einen Vektor zur Expression ergibt.

5. Expressionsvektor gemäß Anspruch 4, wobei der Vektor für die Expression pUCNT ist.

6. Expressionsvektor gemäß Anspruch 5, wobei es sich um pNTRm2 (FERM BP-7333) handelt.

7. Expressionsvektor gemäß Anspruch 5, wobei es sich um pNTRm6 (FERM BP-7332) handelt.

8. Transformante, sich ergebend aus der Transformation eines Wirtsmikroorganismus mit der DNA gemäß Anspruch 1 oder 3.

9. Transformante, sich ergebend aus der Transformation eines Wirtsmikroorganismus mit dem Expressionsvektor gemäß Anspruch 4, 5, 6 oder 7.

10. Transformante gemäß Anspruch 8 oder 9, wobei der Wirtsmikroorganismus ein Stamm von Escherichia coli ist.

11. Transformante gemäß Anspruch 10, wobei der Stamm von Escherichia coli Escherichia coli HB101 ist.

12. Transformante gemäß Anspruch 11, wobei es sich um E. coli HB101 (pNTRm2) (FERM BP-7333) handelt.

13. Transformante gemäß Anspruch 11, wobei es sich um E. coli HB101 (pNTRm6) (FERM BP-7332) handelt.

14. Verfahren zur Erzeugung von Coenzym Q₁₀, das die Kultivierung der Transformante gemäß Anspruch 8, 9, 10, 11, 12 oder 13 in einem Medium und die Gewinnung des Coenzyms Q₁₀, das so gebildet und im Medium angehäuft wurde, umfaßt.

15. DNA gemäß den folgenden (k), (l) oder (m):
(k) eine DNA mit einer Basensequenz wie beschrieben unter SEQ ID Nr. 5;
(l) eine DNA mit einer DNA-Sequenz abgeleitet von der Basensequenz wie dargestellt unter SEQ ID Nr. 5 durch Deletion, Addition, Insertion und/oder Substitution einer Base und ein Protein mit einer Decaprenyldiphosphat-Synthaseaktivität codierend;
(m) eine DNA, die mit einer DNA hybrisidieren kann, umfassend die Basensequenz wie dargestellt unter SEQ ID Nr. 5 unter stringenten Bedingungen;
und ein Protein mit einer Decaprenyldiphosphat-Synthaseaktivität codierend.

16. Protein gemäß den folgenden (n) oder (o):
(n) ein Protein mit einer Aminosäuresequenz wie beschrieben unter SEQ ID Nr. 6;
(0) ein Protein mit einer Aminosäuresequenz abgeleitet von der Aminosäuresequenz wie dargestellt unter SEQ ID Nr. 6 durch Deletion, Addition, Insertion und/oder Substitution einer Aminosäure und mit einer Decaprenyldiphosphat-Synthaseaktivität.

17. DNA, codierend das Protein gemäß Anspruch 16.

18. Expressionsvektor, der sich aus der Insertion der DNA gemäß Anspruch 15 oder 17 in einen Vektor zur Expression ergibt.

19. Expressionsvektor gemäß Anspruch 18, wobei der Vektor für die Expression pUC18 ist.

20. Expressionsvektor gemäß Anspruch 19, wobei es sich um pUCRm3 (FERM BP-7638) handelt.

21. Transformante, sich ergebend aus der Transformation eines Wirtsmikroorganismus mit der DNA gemäß Anspruch 15 oder 17.

22. Transformante, sich ergebend aus der Transformation eines Wirtsmikroorganismus mit dem Expressionsvektor gemäß Anspruch 18, 19 oder 20.

23. Transformante gemäß Anspruch 21 oder 22, wobei der Wirtsmikroorganismus ein Stamm von Escherichia coli ist.

24. Transformante gemäß Anspruch 23, wobei der Stamm von Escherichia coli Escherichia coli DH5α ist.

25. Transformante gemäß Anspruch 24, wobei es sich um E. coli DH5α (pUCRm3) (FERM BP-7638) handelt.

26. Verfahren zur Erzeugung von Coenzym Q₁₀, das die Kultivierung der Transformante gemäß Anspruch 21, 22, 23, 24 oder 25 in einem Medium und die Gewinnung des so gebildeten und im Medium angehäuften Coenzyms Q₁₀ umfaßt.

## Revendications

1. ADN parmi les (d), (e) ou (f) suivants :
(d) un ADN dont la séquence de bases est telle que décrite dans la SEQ ID N° : 3 ;
(e) un ADN ayant une séquence d'ADN dérivée de la séquence de bases représentée par SEQ ID N° 3 par délétion, addition, insertion et/ou substitution d'une base et codant une protéine ayant une activité de décaprényl diphosphate-synthétase ;
(f) un ADN capable de s'hybrider avec un ADN comprenant la séquence de bases représentée par SEQ ID N° : 3 dans des conditions stringentes et
codant une protéine ayant une activité de décaprényl diphosphate-synthétase.

2. Protéine parmi les (i) ou (j) suivants :
(i) une protéine dont un acide aminé est tel que décrit dans la SEQ ID N° : 4 ;
(j) une protéine ayant une séquence d'acides aminés dérivée de la séquence d'acides aminés représentée par SEQ ID N° : 4 par délétion, addition, insertion et/ou substitution d'un résidu d'acide aminé et ayant une activité de décaprényl diphosphate-synthétase.

3. ADN codant la protéine selon la revendication 2.

4. Vecteur d'expression résultant de l'insertion de l'ADN selon la revendication 1 ou 3 dans un vecteur d'expression.

5. Vecteur d'expression selon la revendication 4, dans lequel le vecteur d'expression est pUCNT.

6. Vecteur d'expression selon la revendication 5, qui est pNTRm2 (FERM BP-7333).

7. Vecteur d'expression selon la revendication 5, qui est pNTRm6 (FERM BP-7332).

8. Produit de transformation résultant de la transformation d'un microorganisme hôte avec l'ADN selon la revendication 1 ou 3.

9. Produit de transformation résultant de la transformation d'un microorganisme hôte avec le vecteur d'expression selon la revendication 4, 5, 6 ou 7.

10. Produit de transformation selon la revendication 8 ou 9, dans lequel le microorganisme hôte est une souche d'*Escherichia coli.*

11. Produit de transformation selon la revendication 10, dans lequel la souche *d'Escherichia coli* est *Escherichia coli* HB101.

12. Produit de transformation selon la revendication 11, qui est *E. Coli* HB101 (pNTRm2) (FERM BP-7333).

13. Produit de transformation selon la revendication 11, qui est *E. coli* HB101 (pNTRm6) (FERM BP-7332).

14. Procédé pour produire une coenzyme Q₁₀ qui comprend de cultiver le produit de transformation selon la revendication 8, 9, 10, 11, 12 ou 13 dans un milieu et à récupérer la coenzyme Q₁₀ ainsi formée s'étant accumulée dans le milieu.

15. ADN parmi les (k), (l) ou (m) suivants :
(k) un ADN dont la séquence de bases est telle que décrite dans la SEQ ID N° : 5 ;
(l) un ADN ayant une séquence d'ADN dérivée de la séquence de bases représentée par SEQ ID N° : 5 par délétion, addition, insertion et/ou substitution d'une base et codant une protéine ayant une activité de décaprényl diphosphate-synthétase ;
(m) un ADN capable de s'hybrider avec un ADN comprenant la séquence de bases représentée par SEQ ID N° : 5 dans des conditions stringentes et
codant une protéine ayant une activité de décaprényl diphosphate-synthétase.

16. Protéine parmi les (n) ou (o) suivants :
(n) une protéine dont l'acide aminé est tel que décrit dans la SEQ ID N° : 6 ;
(o) une protéine ayant une séquence d'acides amines dérivée de la séquence d'acides aminés représentée par SEQ ID N° : 6 par délétion, addition, insertion et/ou substitution d'un acide aminé et ayant une activité de décaprényl diphosphate-synthétase.

17. ADN codant la protéine selon la revendication 16.

18. Vecteur d'expression résultant de l'insertion de l'ADN selon la revendication 15 ou 17 dans un vecteur d'expression.

19. Vecteur d'expression selon la revendication 18, dans lequel le vecteur d'expression est pUC18.

20. Vecteur d'expression selon la revendication 19, qui est pUCRm3 (FERM BP-7638).

21. Produit de transformation résultant de la transformation d'un microorganisme hôte avec l'ADN selon la revendication 15 ou 17.

22. Produit de transformation résultant de la transformation d'un microorganisme hôte avec le vecteur d'expression selon la revendication 18, 19 ou 20.

23. Produit de transformation selon la revendication 21 ou 22, dans lequel le microorganisme hôte est une souche d'*Escherichia coli.*

24. Produit de transformation selon la revendication 23, dans lequel la souche d'*Escherichia coli* est *Escherichia coli* DH5α.

25. Produit de transformation selon la revendication 24, qui est *E. Coli* DH5α (pUCRm3) (FERM BP-7638).

26. Procédé pour produire une coenzyme Q₁₀ qui comprend de cultiver le produit de transformation selon la revendication 21, 22, 23, 24 ou 25 dans un milieu et à récupérer la coenzyme Q₁₀ ainsi formée s'étant accumulée dans le milieu.


### SEQUENCE LISTING

<110> KANEKA CORPORATION
<120> Process for producing coenzyme Q₁₀
<130> T640.W01/QX-GT3
<150> JP2000-352940
   <151> 2000-11-20
<160> 6
<210> 1
   <211> 1614
   <212> DNA
   <213> Rhodotorula minuta
<400> 1
   atg atg cac cga caa gct gca tgt aga gtg tgc agt cac act tgc agt  48
   MET MET His Arg Gln Ala Ala Cys Arg Val Cys Ser His Thr Cys Ser
     1               5                  10                  15
   cgc ccg aat gcc ctg ttg gcc ggc ata tat gga cca tca tca gca tca  96
   Arg Pro Asn Ala Leu Leu Ala Gly Ile Tyr Gly Pro Ser Ser Ala Ser
                    20                  25                  30
   tca tcg acg acg acg acg act tca aca tca aga agc aac cac aac aac  144
   Ser Ser Thr Thr Thr Thr Thr Ser Thr Ser Arg Ser Asn His Asn Asn
                35                  40                  45
   agt gta cga ttc aag cat tcg ttg agt aat act agc aaa cca gct gcc  192
   Ser Val Arg Phe Lys His Ser Leu Ser Asn Thr Ser Lys Pro Ala Ala
            50                  55                  60
   aga tcg aca tcc act tcc gct cct gct ctc tct ccc tct tct tca aca   240
   Arg Ser Thr Ser Thr Ser Ala Pro Ala Leu Ser Pro Ser Ser Ser Thr
    65                  70                  75                  80
   agc gat cct caa tct tcc tca tca cca tcc tct tcc tct tct tct tct   288
   Ser Asp Pro Gln Ser Ser Ser Ser Pro Ser Ser Ser Ser Ser Ser Ser
                    85                  90                  95
   tct att ctt cct gac ttt ttg aga tca cca tta tcg tcc tcg tcc tcc   336
   Ser Ile Leu Pro Asp Phe Leu Arg Ser Pro Leu Ser Ser Ser Ser Ser
               100                 105                 110
   tca tcc tcg aca agt agc tct tca tca tca tcg tca agc aat cgc tcc   384
   Ser Ser Ser Thr Ser Ser Ser Ser Ser Ser Ser Ser Ser Asn Arg Ser
           115                 120                 125
   aaa aac act aac agc aat aca atc ttc gac cca ctc caa ctc gta ggc   432
   Lys Asn Thr Asn Ser Asn Thr Ile Phe Asp Pro Leu Gln Leu Val Gly
       130                 135                 140
   aat gaa ctg tca agt ctc cgg tca aac gtc caa gcc cta ctg gga tca   480
   Asn Glu Leu Ser Ser Leu Arg Ser Asn Val Gln Ala Leu Leu Gly Ser
   145                 150                 155                 160
   ggt cat ccc gcc cta gac acg ata gca aag tac tac ttc caa gcg gag   528
   Gly His Pro Ala Leu Asp Thr Ile Ala Lys Tyr Tyr Phe Gln Ala Glu
                   165                 170                 175
   ggc aaa cat att cgg cct atg atc gtt ctt ctc atg tcc caa gcc aca   576
   Gly Lys His Ile Arg Pro MET Ile Val Leu Leu MET Ser Gln Ala Thr
               180                 185                 190
   aac ggt cta gcg ccc ggg ttt gaa gaa cgc tca aaa ttg gaa cta tca   624
   Asn Gly Leu Ala Pro Gly Phe Glu Glu Arg Ser Lys Leu Glu Leu Ser
   195 200 205
   ggt cgg aaa cag act gat ccc tcc agg tca atc aat gat cct ctc gaa 672
   Gly Arg Lys Gln Thr Asp Pro Ser Arg Ser Ile Asn Asp Pro Leu Glu
   210 215 220
   gtg aaa gca gat gag ata ctc aac gat tcg aat ccc tct tcg ttc gct 720
   Val Lys Ala Asp Glu Ile Leu Asn Asp Ser Asn Pro Ser Ser Phe Ala
   225 230 235 240
   gcg agc tcc tct tcg ccg ctc gat agc atg ccg tcc acg tcg aat gtc 768
   Ala Ser Ser Ser Ser Pro Leu Asp Ser MET Pro Ser Thr Ser Asn Val
   245 250 255
   cta ccc tcg caa cga cgc ctc gcg gaa atc acc gaa atg atc cac gta 816
   Leu Pro Ser Gln Arg Arg Leu Ala Glu Ile Thr Glu MET Ile His Val
   260 265 270
   gct tcg cta ttg cac gac gat gtc ata gac ggt tca gcc atg agg aga 864
   Ala Ser Leu Leu His Asp Asp Val Ile Asp Gly Ser Ala MET Arg Arg
   275 280 285
   gca caa gcg tcc gcc ccc gct gca ttc ggg aac aag atc tcg gtg ctg 912
   Ala Gln Ala Ser Ala Pro Ala Ala Phe Gly Asn Lys Ile Ser Val Leu
   290 295 300
   ggc ggg gat ttc ctc ctc gct cgt gct tcg ctg tac ctc tcc cga cta 960
   Gly Gly Asp Phe Leu Leu Ala Arg Ala Ser Leu Tyr Leu Ser Arg Leu
   305 310 315 320
   ggg agc aac gag gtc gtc gag cta gta gcc tcc gtg cta gct aat cta 1008
   Gly Ser Asn Glu Val Val Glu Leu Val Ala Ser Val Leu Ala Asn Leu
   325 330 335
   gta gag ggc gaa gtc atg cag atc aag gga aat gct cct gaa agc aat 1056
   Val Glu Gly Glu Val MET Gln Ile Lys Gly Asn Ala Pro Glu Ser Asn
   340 345 350
   gca agc gga agc aaa gag gta gca gtg cac aga ttg acc ccg gaa att 1104
   Ala Ser Gly Ser Lys Glu Val Ala Val His Arg Leu Thr Pro Glu Ile
   355 360 365
   ttc gaa cat tat atg aag aag aca tac ttg aag acc gca agt ctc atc 1152
   Phe Glu His Tyr MET Lys Lys Thr Tyr Leu Lys Thr Ala Ser Leu Ile
   370 375 380
   gcg aaa tcg aca aga gcg acc act atc ctc ggt gga gca ggc gag aaa 1200
   Ala Lys Ser Thr Arg Ala Thr Thr Ile Leu Gly Gly Ala Gly Glu Lys
   385 390 395 400
   cag ggg tgg ata gag ggc gag cgc ata aaa gac att gcg tac tcg tac 1248
   Gln Gly Trp Ile Glu Gly Glu Arg Ile Lys Asp Ile Ala Tyr Ser Tyr
   405 410 415
   ggt cgc aat cta ggt att gct ttc cag ctc gtc gac gat cta cta gat 1296
   Gly Arg Asn Leu Gly Ile Ala Phe Gln Leu Val Asp Asp Leu Leu Asp
   420 425 430
   ttc aca gct aca gac gcg caa ttc ggc aag ccc tca cag ggt gca gat 1344
   Phe Thr Ala Thr Asp Ala Gln Phe Gly Lys Pro Ser Gln Gly Ala Asp
   435 440 445
   ctg aag ctc ggt ctc gca act gcg ccc gcg ctg tac gca tgg gaa gag 1392
   Leu Lys Leu Gly Leu Ala Thr Ala Pro Ala Leu Tyr Ala Trp Glu Glu
   450 455 460
   ttc ccg gag atg ggc cag atg att ctc cgc aag ttt gag aac gaa ggc 1440
   Phe Pro Glu MET Gly Gln MET Ile Leu Arg Lys Phe Glu Asn Glu Gly
   465 470 475 480
   gat gtc gaa act gcc agg aat cta gta aga aag tca gct gga ccg gaa 1488
   Asp Val Glu Thr Ala Arg Asn Leu Val Arg Lys Ser Ala Gly Pro Glu
   485 490 495
   aag acc gtg aaa ttg gcg gaa aaa cat gcc gca ctc gca atg gag gcc 1536
   Lys Thr Val Lys Leu Ala Glu Lys His Ala Ala Leu Ala MET Glu Ala
   500 505 510
   ctg cag gga ttg ccg gag tcg gac gct aga gaa gcg ctc gaa ggc ctg 1584
   Leu Gln Gly Leu Pro Glu Ser Asp Ala Arg Glu Ala Leu Glu Gly Leu
   515 520 525
   acc aag act gtg ctc aac cga aca aag tag 1614
   Thr Lys Thr Val Leu Asn Arg Thr Lys
   530 535
<210> 2
   <211> 537
   <212> PRT
   <213> Rhodotorula minuta
<400> 2
   MET MET His Arg Gln Ala Ala Cys Arg Val Cys Ser His Thr Cys
   1 5 10 15
   Ser Arg Pro Asn Ala Leu Leu Ala Gly Ile Tyr Gly Pro Ser Ser
   20 25 30
   Ala Ser Ser Ser Thr Thr Thr Thr Thr Ser Thr Ser Arg Ser Asn
   35 40 45
   His Asn Asn Ser Val Arg Phe Lys His Ser Leu Ser Asn Thr Ser
   50 55 60
   Lys Pro Ala Ala Arg Ser Thr Ser Thr Ser Ala Pro Ala Leu Ser
   65 70 75
   Pro Ser Ser Ser Thr Ser Asp Pro Gln Ser Ser Ser Ser Pro Ser
   80 85 90
   Ser Ser Ser Ser Ser Ser Ser Ile Leu Pro Asp Phe Leu Arg Ser
   95 100 105
   Pro Leu Ser Ser Ser Ser Ser Ser Ser Ser Thr Ser Ser Ser Ser
   110 115 120
   Ser Ser Ser Ser Ser Asn Arg Ser Lys Asn Thr Asn Ser Asn Thr
   125 130 135
   Ile Phe Asp Pro Leu Gln Leu Val Gly Asn Glu Leu Ser Ser Leu
   140 145 150
   Arg Ser Asn Val Gln Ala Leu Leu Gly Ser Gly His Pro Ala Leu
   155 160 165
   Asp Thr Ile Ala Lys Tyr Tyr Phe Gln Ala Glu Gly Lys His Ile
   170 175 180
   Arg Pro MET Ile Val Leu Leu MET Ser Gln Ala Thr Asn Gly Leu
   185 190 195
   Ala Pro Gly Phe Glu Glu Arg Ser Lys Leu Glu Leu Ser Gly Arg
   200 205 210
   Lys Gln Thr Asp Pro Ser Arg Ser Ile Asn Asp Pro Leu Glu Val
   215 220 225
   Lys Ala Asp Glu Ile Leu Asn Asp Ser Asn Pro Ser Ser Phe Ala
   230 235 240
   Ala Ser Ser Ser Ser Pro Leu Asp Ser MET Pro Ser Thr Ser Asn
   245 250 255
   Val Leu Pro Ser Gln Arg Arg Leu Ala Glu Ile Thr Glu MET Ile
   260 265 270
   His Val Ala Ser Leu Leu His Asp Asp Val Ile Asp Gly Ser Ala
   275 280 285
   MET Arg Arg Ala Gln Ala Ser Ala Pro Ala Ala Phe Gly Asn Lys
   290 295 300
   Ile Ser Val Leu Gly Gly Asp Phe Leu Leu Ala Arg Ala Ser Leu
   305 310 315
   Tyr Leu Ser Arg Leu Gly Ser Asn Glu Val Val Glu Leu Val Ala
   320 325 330
   Ser Val Leu Ala Asn Leu Val Glu Gly Glu Val MET Gln Ile Lys
   335 340 345
   Gly Asn Ala Pro Glu Ser Asn Ala Ser Gly Ser Lys Glu Val Ala
   350 355 360
   Val His Arg Leu Thr Pro Glu Ile Phe Glu His Tyr MET Lys Lys
   365 370 375
   Thr Tyr Leu Lys Thr Ala Ser Leu Ile Ala Lys Ser Thr Arg Ala
   380 385 390
   Thr Thr Ile Leu Gly Gly Ala Gly Glu Lys Gln Gly Trp Ile Glu
   395 400 405
   Gly Glu Arg Ile Lys Asp Ile Ala Tyr Ser Tyr Gly Arg Asn Leu
   410 415 420
   Gly Ile Ala Phe Gln Leu Val Asp Asp Leu Leu Asp Phe Thr Ala
   425 430 435
   Thr Asp Ala Gln Phe Gly Lys Pro Ser Gln Gly Ala Asp Leu Lys
   440 445 450
   Leu Gly Leu Ala Thr Ala Pro Ala Leu Tyr Ala Trp Glu Glu Phe
   455 460 465
   Pro Glu MET Gly Gln MET Ile Leu Arg Lys Phe Glu Asn Glu Gly
   470 475 480
   Asp Val Glu Thr Ala Arg Asn Leu Val Arg Lys Ser Ala Gly Pro
   485 490 495
   Glu Lys Thr Val Lys Leu Ala Glu Lys His Ala Ala Leu Ala MET
   500 505 510
   Glu Ala Leu Gln Gly Leu Pro Glu Ser Asp Ala Arg Glu Ala Leu
   515 520 525
   Glu Gly Leu Thr Lys Thr Val Leu Asn Arg Thr Lys
   530 535
<210> 3
   <211> 1278
   <212> DNA
   <213> Rhodotorula minuta
<400> 3
   atg atg cac cga caa gct gca tgt aga gtg tgc agt cac act tgc agt 48
   MET MET His Arg Gln Ala Ala Cys Arg Val Cys Ser His Thr Cys Ser
   1 5 10 15
   cgc ccg aat gcc ctc aat aca atc ttc gac cca ctc caa ctc gta ggc 96
   Arg Pro Asn Ala Leu Asn Thr Ile Phe Asp Pro Leu Gln Leu Val Gly
   20 25 30
   aat gaa ctg tca agt ctc cgg tca aac gtc caa gcc cta ctg gga tca 144
   Asn Glu Leu Ser Ser Leu Arg Ser Asn Val Gln Ala Leu Leu Gly Ser
   35 40 45
   ggt cat ccc gcc cta gac acg ata gca aag tac tac ttc caa gcg gag 192
   Gly His Pro Ala Leu Asp Thr Ile Ala Lys Tyr Tyr Phe Gln Ala Glu
   50 55 60
   ggc aaa cat att cgg cct atg atc gtt ctt ctc atg tcc caa gcc aca 240
   Gly Lys His Ile Arg Pro MET Ile Val Leu Leu MET Ser Gln Ala Thr
   65 70 75 80
   aac ggt cta gcg ccc ggg ttt gaa gaa cgc tca aaa ttg gaa cta tca 288
   Asn Gly Leu Ala Pro Gly Phe Glu Glu Arg Ser Lys Leu Glu Leu Ser
   85 90 95
   ggt cgg aaa cag act gat ccc tcc agg tca atc aat gat cct ctc gaa 336
   Gly Arg Lys Gln Thr Asp Pro Ser Arg Ser Ile Asn Asp Pro Leu Glu
   100 105 110
   gtg aaa gca gat gag ata ctc aac gat tcg aat ccc tct tcg ttc gct 384
   Val Lys Ala Asp Glu Ile Leu Asn Asp Ser Asn Pro Ser Ser Phe Ala
   115 120 125
   gcg agc tcc tct tcg ccg ctc gat agc atg ccg tcc acg tcg aat gtc 432
   Ala Ser Ser Ser Ser Pro Leu Asp Ser MET Pro Ser Thr Ser Asn Val
   130 135 140
   cta ccc tcg caa cga cgc ctc gcg gaa atc acc gaa atg atc cac gta 480
   Leu Pro Ser Gln Arg Arg Leu Ala Glu Ile Thr Glu MET Ile His Val
   145 150 155 160
   gct tcg cta ttg cac gac gat gtc ata gac ggt tca gcc atg agg aga 528
   Ala Ser Leu Leu His Asp Asp Val Ile Asp Gly Ser Ala MET Arg Arg
   165 170 175
   gca caa gcg tcc gcc ccc gct gca ttc ggg aac aag atc tcg gtg ctg 576
   Ala Gln Ala Ser Ala Pro Ala Ala Phe Gly Asn Lys Ile Ser Val Leu
   180 185 190
   ggc ggg gat ttc ctc ctc gct cgt gct tcg ctg tac ctc tcc cga cta 624
   Gly Gly Asp Phe Leu Leu Ala Arg Ala Ser Leu Tyr Leu Ser Arg Leu
   195 200 205
   ggg agc aac gag gtc gtc gag cta gta gcc tcc gtg cta gct aat cta 672
   Gly Ser Asn Glu Val Val Glu Leu Val Ala Ser Val Leu Ala Asn Leu
   210 215 220
   gta gag ggc gaa gtc atg cag atc aag gga aat gct cct gaa agc aat 720
   Val Glu Gly Glu Val MET Gln Ile Lys Gly Asn Ala Pro Glu Ser Asn
   225 230 235 240
   gca agc gga agc aaa gag gta gca gtg cac aga ttg acc ccg gaa att 768
   Ala Ser Gly Ser Lys Glu Val Ala Val His Arg Leu Thr Pro Glu Ile
   245 250 255
   ttc gaa cat tat atg aag aag aca tac ttg aag acc gca agt ctc atc 816
   Phe Glu His Tyr MET Lys Lys Thr Tyr Leu Lys Thr Ala Ser Leu Ile
   260 265 270
   gcg aaa tcg aca aga gcg acc act atc ctc ggt gga gca ggc gag aaa 864
   Ala Lys Ser Thr Arg Ala Thr Thr Ile Leu Gly Gly Ala Gly Glu Lys
   275 280 285
   cag ggg tgg ata gag ggc gag cgc ata aaa gac att gcg tac tcg tac   912
   Gln Gly Trp Ile Glu Gly Glu Arg Ile Lys Asp Ile Ala Tyr Ser Tyr
       290                 295                 300
   ggt cgc aat cta ggt att gct ttc cag ctc gtc gac gat cta cta gat   960
   Gly Arg Asn Leu Gly Ile Ala Phe Gln Leu Val Asp Asp Leu Leu Asp
   305                 310                 315                 320
   ttc aca gct aca gac gcg caa ttc ggc aag ccc tca cag ggt gca gat   1008
   Phe Thr Ala Thr Asp Ala Gln Phe Gly Lys Pro Ser Gln Gly Ala Asp
                   325                 330                 335
   ctg aag ctc ggt ctc gca act gcg ccc gcg ctg tac gca tgg gaa gag   1056
   Leu Lys Leu Gly Leu Ala Thr Ala Pro Ala Leu Tyr Ala Trp Glu Glu
               340                 345                 350
   ttc ccg gag atg ggc cag atg att ctc cgc aag ttt gag aac gaa ggc   1104
   Phe Pro Glu MET Gly Gln MET Ile Leu Arg Lys Phe Glu Asn Glu Gly
           355                 360                 365
   gat gtc gaa act gcc agg aat cta gta aga aag tca gct gga ccg gaa   1152
   Asp Val Glu Thr Ala Arg Asn Leu Val Arg Lys Ser Ala Gly Pro Glu
       370                 375                 380
   aag acc gtg aaa ttg gcg gaa aaa cat gcc gca ctc gca atg gag gcc   1200
   Lys Thr Val Lys Leu Ala Glu Lys His Ala Ala Leu Ala MET Glu Ala
   385                 390                 395                 400
   ctg cag gga ttg ccg gag tcg gac gct aga gaa gcg ctc gaa ggc ctg   1248
   Leu Gln Gly Leu Pro Glu Ser Asp Ala Arg Glu Ala Leu Glu Gly Leu
                   405                 410                 415
   acc aag act gtg ctc aac cga aca aag tag                           1278
   Thr Lys Thr Val Leu Asn Arg Thr Lys
               420                 425
<210> 4
   <211> 425
   <212> PRT
   <213> Rhodotorula
<400> 4
   MET MET His Arg Gln Ala Ala Cys Arg Val Cys Ser His Thr Cys
   1               5                   10                  15
   Ser Arg Pro Asn Ala Leu Asn Thr Ile Phe Asp Pro Leu Gln Leu
                   20                  25                  30
   Val Gly Asn Glu Leu Ser Ser Leu Arg Ser Asn Val Gln Ala Leu
                   35                  40                  45
   Leu Gly Ser Gly His Pro Ala Leu Asp Thr Ile Ala Lys Tyr Tyr
                   50                  55                  60
   Phe Gln Ala Glu Gly Lys His Ile Arg Pro MET Ile Val Leu Leu
                   65                  70                  75
   MET Ser Gln Ala Thr Asn Gly Leu Ala Pro Gly Phe Glu Glu Arg
                   80                  85                  90
   Ser Lys Leu Glu Leu Ser Gly Arg Lys Gln Thr Asp Pro Ser Arg
                   95                  100                 105
   Ser Ile Asn Asp Pro Leu Glu Val Lys Ala Asp Glu Ile Leu Asn
                   110                 115                 120
   Asp Ser Asn Pro Ser Ser Phe Ala Ala Ser Ser Ser Ser Pro Leu
                   125                 130                 135
   Asp Ser MET Pro Ser Thr Ser Asn Val Leu Pro Ser Gln Arg Arg
   140 145 150
   Leu Ala Glu Ile Thr Glu MET Ile His Val Ala Ser Leu Leu His
   155 160 165
   Asp Asp Val Ile Asp Gly Ser Ala MET Arg Arg Ala Gln Ala Ser
   170 175 180
   Ala Pro Ala Ala Phe Gly Asn Lys Ile Ser Val Leu Gly Gly Asp
   185 190 195
   Phe Leu Leu Ala Arg Ala Ser Leu Tyr Leu Ser Arg Leu Gly Ser
   200 205 210
   Asn Glu Val Val Glu Leu Val Ala Ser Val Leu Ala Asn Leu Val
   215 220 225
   Glu Gly Glu Val MET Gln Ile Lys Gly Asn Ala Pro Glu Ser Asn
   230 235 240
   Ala Ser Gly Ser Lys Glu Val Ala Val His Arg Leu Thr Pro Glu
   245 250 255
   Ile Phe Glu His Tyr MET Lys Lys Thr Tyr Leu Lys Thr Ala Ser
   260 265 270
   Leu Ile Ala Lys Ser Thr Arg Ala Thr Thr Ile Leu Gly Gly Ala
   275 280 285
   Gly Glu Lys Gln Gly Trp Ile Glu Gly Glu Arg Ile Lys Asp Ile
   290 295 300
   Ala Tyr Ser Tyr Gly Arg Asn Leu Gly Ile Ala Phe Gln Leu Val
   305 310 315
   Asp Asp Leu Leu Asp Phe Thr Ala Thr Asp Ala Gln Phe Gly Lys
   320 325 330
   Pro Ser Gln Gly Ala Asp Leu Lys Leu Gly Leu Ala Thr Ala Pro
   335 340 345
   Ala Leu Tyr Ala Trp Glu Glu Phe Pro Glu MET Gly Gln MET Ile
   350 355 360
   Leu Arg Lys Phe Glu Asn Glu Gly Asp Val Glu Thr Ala Arg Asn
   365 370 375
   Leu Val Arg Lys Ser Ala Gly Pro Glu Lys Thr Val Lys Leu Ala
   380 385 390
   Glu Lys His Ala Ala Leu Ala MET Glu Ala Leu Gln Gly Leu Pro
   395 400 405
   Glu Ser Asp Ala Arg Glu Ala Leu Glu Gly Leu Thr Lys Thr Val
   410 415 420
   Leu Asn Arg Thr Lys
   425
<210> 5
   <211> 1212
   <212> DNA
   <213> Rhodotorula minuta
<400> 5
<210> 6
   <211> 403
   <212> PRT
   <213> Rhodotorula minuta
<400> 6
   Met Ile Phe Asp Pro Leu Gln Leu Val Gly Asn Glu Leu Ser Ser
   1 5 10 15
   Leu Arg Ser Asn Val Gln Ala Leu Leu Gly Ser Gly His Pro Ala
   20 25 30
   Leu Asp Thr Ile Ala Lys Tyr Tyr Phe Gln Ala Glu Gly Lys His
   35 40 45
   Ile Arg Pro Met Ile Val Leu Leu Met Ser Gln Ala Thr Asn Gly
   50 55 60
   Leu Ala Pro Gly Phe Glu Glu Arg Ser Lys Leu Glu Leu Ser Gly
   65 70 75
   Arg Lys Gln Thr Asp Pro Ser Arg Ser Ile Asn Asp Pro Leu Glu
   80 85 90
   Val Lys Ala Asp Glu Ile Leu Asn Asp Ser Asn Pro Ser Ser Phe
   95 100 105
   Ala Ala Ser Ser Ser Ser Pro Leu Asp Ser Met Pro Ser Thr Ser
   110 115 120
   Asn Val Leu Pro Ser Gln Arg Arg Leu Ala Glu Ile Thr Glu Met
   125 130 135
   Ile His Val Ala Ser Leu Leu His Asp Asp Val Ile Asp Gly Ser
   140 145 150
   Ala Met Arg Arg Ala Gln Ala Ser Ala Pro Ala Ala Phe Gly Asn
   155 160 165
   Lys Ile Ser Val Leu Gly Gly Asp Phe Leu Leu Ala Arg Ala Ser
   170 175 180
   Leu Tyr Leu Ser Arg Leu Gly Ser Asn Glu Val Val Glu Leu Val
   185 190 195
   Ala Ser Val Leu Ala Asn Leu Val Glu Gly Glu Val Met Gln Ile
   200 205 210
   Lys Gly Asn Ala Pro Glu Ser Asn Ala Ser Gly Ser Lys Glu Val
   215 220 225
   Ala Val His Arg Leu Thr Pro Glu Ile Phe Glu His Tyr Met Lys
   230 235 240
   Lys Thr Tyr Leu Lys Thr Ala Ser Leu Ile Ala Lys Ser Thr Arg
   245 250 255
   Ala Thr Thr Ile Leu Gly Gly Ala Gly Glu Lys Gln Gly Trp Ile
   260 265 270
   Glu Gly Glu Arg Ile Lys Asp Ile Ala Tyr Ser Tyr Gly Arg Asn
   275 280 285
   Leu Gly Ile Ala Phe Gln Leu Val Asp Asp Leu Leu Asp Phe Thr
   290 295 300
   Ala Thr Asp Ala Gln Phe Gly Lys Pro Ser Gln Gly Ala Asp Leu
   305 310 315
   Lys Leu Gly Leu Ala Thr Ala Pro Ala Leu Tyr Ala Trp Glu Glu
   320 325 330
   Phe Pro Glu Met Gly Gln Met Ile Leu Arg Lys Phe Glu Asn Glu
   335 340 345
   Gly Asp Val Glu Thr Ala Arg Asn Leu Val Arg Lys Ser Ala Gly
   350 355 360
   Pro Glu Lys Thr Val Lys Leu Ala Glu Lys His Ala Ala Leu Ala
   365 370 375
   MET Glu Ala Leu Gln Gly Leu Pro Glu Ser Asp Ala Arg Glu Ala
   380 385 390
   Leu Glu Gly Leu Thr Lys Thr Val Leu Asn Arg Thr Lys
   395 400 403